# EUROPÄISCHE PATENTSCHRIFT

(11) **EP 0 841 875 B1**
(45) Veröffentlichungstag und Bekanntmachung des Hinweises auf die Patenterteilung: **02.06.1999**
(21) Anmeldenummer: 96927575.9
(22) Anmeldetag: 22.07.1996
(51) Int. Cl.: A61B 17/16

(54) **BOHRKOPF ZUM AUFBOHREN VON KNOCHENKANÄLEN**
BORE HEAD FOR BORING BONE CHANNELS
TETE DE PERCAGE DE CANAUX DANS DES OS

(30) Priorität: 22.07.1995 DE 29511872 U
(43) Veröffentlichungstag der Anmeldung: 20.05.1998
(73) Patentinhaber: Howmedica GmbH, D-24232 Schönkirchen (DE)
(72) Erfinder: HARDER, Hans, Erich, D-24253 Probsteierhagen (DE)
(74) Vertreter: Patentanwälte Hauck, Graalfs, Wehnert, Döring, Siemons
(86) Internationale Anmeldenummer: EP9603230
(87) Internationale Veröffentlichungsnummer: WO9703617

(56) Entgegenhaltungen:
- EP-A- 0 440 371
- EP-A- 0 508 710
- GB-A- 219 479

## Beschreibung

Die Erfindung bezieht sich auf einen Bohrkopf zum Aufbohren von Knochenkanälen nach den, Oberbegriff des Anspruchs 1.

Insbesondere vor dem Einsetzen von Implantaten in Röhrenknochen werden die Knochenkanäle zunächst aufgebohrt, damit das Implantat eingeführt bzw. eingeschlagen werden kann. Zum Aufbohren wird zumeist ein Spiralbohrkopf verwendet, der einen relativ kurzen Schaft aufweist, der mit einem flexiblen Antriebsschaft koppelbar ist und der von einer geeigneten Antriebsmaschine in Drehung versetzt wird. Der flexible Antriebsschaft und der Bohrkopf weisen eine Durchbohrung auf Sie dient zur Aufnahme eines Führungsspießes, der zuvor in den Knochen eingeschlagen worden ist. Auf diese Weise wird eine Führung für den Bohrkopferhalten und verhindert, daß die Knochenwandung seitlich durchbohrt wird.

Bei dem Aufbohren von Knochenkanälen besteht die Gefahr, daß unnötige Verletzungen auftreten. Insbesondere wenn der Druck und die Temperatur im Markraum stark anwachsen, besteht zudem die Gefahr von Fettembolien. Diese müssen in jedem Fall vermieden werden.

Aus EP-A0 440 371 ist ein Räumwerkzeug bekannt geworden, dessen Bohrkopf eine Reihe von gedrallten Zähnen aufweist, die zwischen sich Spankammern bilden. Die Zähne sind in der Außenkontur gerundet, wodurch nur der zwischen den Enden liegende mittlere Bereich eine Zerspanung bewirkt. Der Bohrkopf ist mit einem flexiblen Schaft verbunden, der als biegsame Welle wirkt. Aus der gleichen Druckschrift ist auch ein Räumwerkzeug bekannt geworden, das eine Reihe von Zähnen am Bohrkopf aufweist, die in einen Anschnitt auslaufen und in diesem Bereich die Hauptschneidkanten bilden. Die Nebenschneidkanten sind gerade und liegen auf einem Zylinder, dessen Achse mit der Achse des Bohrkopfes zusammenfällt.

Ein Räumwerkzeug der eingangs genannten Art ist aus der EP A-0 508 710 bekannt geworden. Es weist vier im 90°-Umfangsabstand angeordnete Zähne auf mit einem Anschnitt, in dem die Hauptschneidkanten gebildet sind. Die Nebenschneidkanten liegen auf einem Zylinder, dessen Achse mit der Bohrung zusammenfällt. Die Länge der Nebenschneidkanten ist kleiner als der Durchmesser des Zylinders. Der bekannte Bohrkopf ist mit einem starren Schaft verbunden und dient vor allen Dingen zum Aufbohren geringerer Längen im Knochen.

Der Erfindung liegt die Aufgabe zugrunde, ein Räumwerkzeug zum Aufbohren von Knochenkanälen zu schaffen, das einen relativ raschen Vorschub erlaubt bei gleichzeitig guter Spanabfuhr und geringem Druck.

Diese Aufgabe wird erfindungsgemäß gelöst durch die Merkmale des Anspruchs 1.

Der erfindungsgemäße Bohrkopf weist vier im 90°-Umfangsabstand angeordnete schraubenlinienförmig verlaufende Zähne auf die einen kleinen Drallwinkel aufweisen. Zum Beispiel liegt der Drallwinkel im Bereich von 15°. Der Anschnitt weist einen Winkel zur Längsachse von etwa 45° auf (Spitzenwinkel etwa 90°). Es hat sich gezeigt, daß ein derartiger Anschnitt besonders günstig ist.

Die Nebenschneidkanten außen an den Zähnen liegen annähernd auf einem Zylinder, dessen Achse mit der Achse der Bohrung zusammenfällt. Die Nebenschneidkanten erstrecken sich somit annähernd auf einer Geraden, die annähernd gleichen Abstand zur Achse längs der Bohrung über ihre Länge aufweist Eine derartige Ausbildung der Zähne ermöglicht eine gute Führung im Knochenkanal unabhängig vom Führungsspieß. Es wird die Gefahr vermieden, daß der Bohrkopf sich seitlich zu weit in die Knochenwandung eingräbt.

Ein weiteres Merkmal der Erfindung liegt darin, daß der Grund der Spankammer zwischen den Zähnen oder die Spannut radial sehr nahe an der Bohrung liegt. Es ist nur so viel Material zur Bohrung hin stehen gelassen, daß der Bohrkopf eine ausreichende Stabilität erhält. Beispielsweise liegt der Grund der Spankammer in einem radialen Abstand zur Bohrungsachse, der etwa dem Außenradius des Schaftes des Bohrkopfs entspricht. Es ist jedoch denkbar, den Grund radial noch näher an die Bohrung heranzuführen, wenn ein entsprechender Übergang von der Spankammer zum Schaft hin geschaffen wird, so daß ein Spanstau nicht auftritt. Die auf diese Weise geschaffenen relativ großen Spankammern oder Spannuten ermöglichen eine hervorragende Spanabfuhr.

Schließlich ist erfindungsgemäß im Anschnittbereich an den die Spankammern begrenzenden Flächen der Zähne ein Anschliff vorgenommen derart, daß ein relativ scharfer Rand von den Enden der Zähne und den dazwischenliegenden Spankammern zur Bohrung hin gebildet ist. Dieses Merkmal bedingt eine Anschärfung bzw. Anspitzung des Anschnitts, wodurch ein wirksames Eindringen und "Einfräsen" in den Knochenkanal ermöglicht ist, auch bei engen Markkanälen.

Da Gefahr bestehen könnte, daß der Führungsspieß den gebildeten scharfen Rand der Bohrung beschädigt, sieht eine Ausgestaltung der Erfindung vor, daß die Bohrung im vorderen Ende eine konische Ansenkung aufweist. Auf diese Weise kann eine gewisse Verkantung aufgrund von Toleranzen zwischen Bohrkopf und Führungsspieß stattfinden, ohne daß der gleichwohl verbleibende scharfe Rand eine Beschädigung erleidet.

Nach einer weiteren Ausgestaltung der Erfindung ist die Außenkontur der Zähne am hinteren Ende mit einer Rundung versehen, die dann auf den Schaft des Bohrkopfes trifft. Der Radius der Rundung wird relativ groß gewählt, um ein Hinterschneiden beim Bohren im Kanal zu verhindern.

Die Länge der Neben-Schneidkante, die sich, wie bereits beschrieben, auf einer Zylinderfläche befindet, darf nicht zu groß gewählt werden, weil dann die Gefahr besteht, daß es zu Hemmungen kommt. Daher ist die Länge dieser Schneidkante auf jeden Fall kleiner als der Durchmesser des Zylinders.

Der Spanwinkel an Haupt- und Nebenschneidkante bewegt sich vorzugsweise im Bereich von 20 bis 22°. Die Spanfläche wird nach einer Ausgestaltung der Erfindung von einer durchgehenden Hohlkehle gebildet, die sich bis zum Anschliff erstreckt. Zusammen mit der relativ tiefen Spankammer wird dadurch ein erwünschtes Ausschälen des Knochenmaterials erhalten bei gleichzeitig guter Spanabfuhr.

Der Freiwinkel der Hauptschneidkante liegt nach einer weiteren Ausgestaltung der Erfindung etwa im Bereich von 13 bis 17°, wobei sich an den Neben-Schneidkanten zwei im Winkel unterschiedliche Freiflächen ausgebildet sein können. Die erste sich an die Schneidkante anschließende Freifläche hat zum Beispiel einen Winkel von 13 bis 15°, während die sich daran anschließende Freifläche einen Winkel von 15 bis 17° aufweist.

Die Erfindung wird nachfolgend anhand von Zeichnungen näher erläutert.
- Fig. 1a: zeigt teilweise in Seitenansicht, teilweise im Schnitt einen Bohrkopf nach der Erfindung, teilweise vor dem letzten Fertigungsgang.
- Fig. 1b: zeigt ein Teil des Bohrkopfs nach Fig. 4 in Seitenansicht
- Fig. 2: zeigt eine Endansicht des Bohrkopfs nach Fig. 1 in Richtung Pfeil 2, wobei jedoch die Ausbildung nur der oberen Hälfte wiedergegeben ist, d.h. vor dem letzten Fertigungsgang.
- Fig. 3: zeigt eine ähnliche Darstellung wie Fig. 1, jedoch nach endgültiger Fertigstellung.
- Fig. 4: zeigt eine Endansicht des Bohrkopfs nach Fig. 3 in Richtung Pfeil 4.
- Fig. 5: zeigt einen Schnitt durch einen Zahn des Bohrkopfs nach Fig. 3 im radial äußeren Bereich.

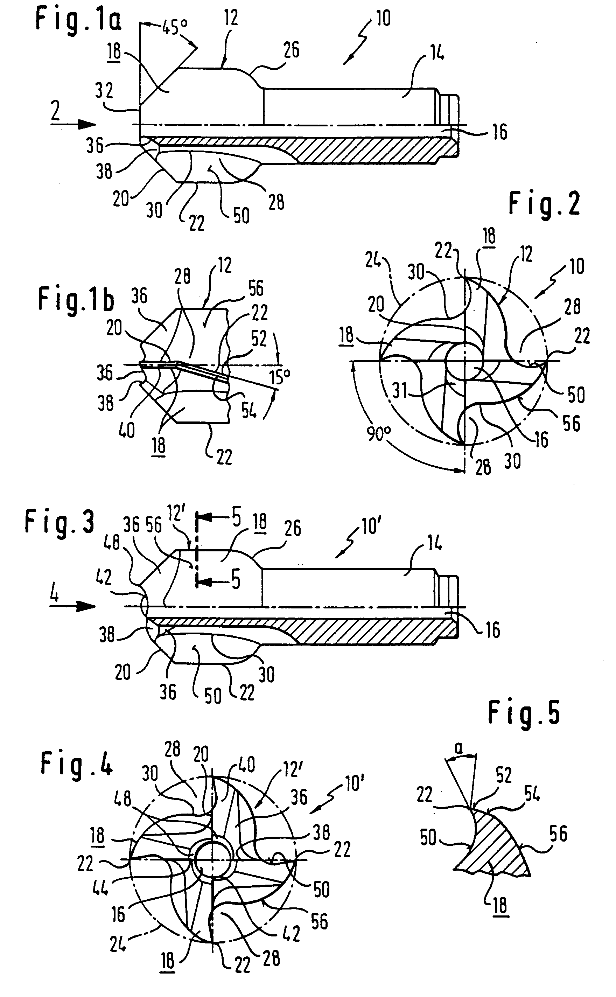

Das in Fig. 1 gezeigte Werkzeug 10 besteht aus dem eigentlichen Bohrkopf 12 und einem zylindrischen Schaft 14. Schaft 14 und Kopf 12 sind mit einer mittigen Durchbohrung 16 geformt, von der nur die untere Hälfte in Fig. 1 zu erkennen ist. Die Bohrung hat zum Beispiel einen Innendurchmesser von 3,6 mm.

Es sei erwähnt, daß die obere Hälfte von Fig. 1 einen Zwischenbearbeitungszustand zeigt, während die untere Hälfte den Endbearbeitungszustand wiedergibt.

Der Bohrkopf 12 weist vier im 90°-Abstand angeordnete und sich annähernd achsparallel und radial nach außen erstreckende Zähne 28 auf, die im Anschnittbereich eine Haupt-Schneidkante 20 mit einem Winkel von 45° zur Längsachse (Fig. 1) aufweisen. Die Neben-Schneidkanten 22 der Zähne 18 liegen auf der Fläche eines Zylinders, dessen Achse mit der Achse der Bohrung 16 konform ist. Ein derartiger Zylinder ist in Fig. 2 bei 24 strichpunktiert angegeben. Die Zähne 18 haben einen leicht schraubenförmigen Verlauf mit einem Drallwinkel von zum Beispiel etwa 15° (Fig. 1b). Am hinteren Ende geht die Außenkontur der Zähne bogenförmig in den Schaft über, wie bei 26 angedeutet. Der Bogen 26 weist einen relativ großen Radius auf.

Zwischen den Zähnen 18 sind nutenförmige Spankammern 28 gebildet, deren Grund 30, der mit einem relativ kleinen Radius geformt ist, radial gesehen relativ nahe an der Bohrung 16 liegt, mit anderen Worten, die Materialdicke zwischen Grund 30 und Bohrung 16 ist relativ klein. Die Spankammern haben daher einen relativ großen Querschnitt, können daher viele Späne aufnehmen und abführen.

Bei der Fertigung wird der Bohrkopf 12 zunächst so geformt, daß sich an der Spitze vier Ringflächenabschnitte 31 bilden, die im Bereich des vorderen Endes der Zähne 18 annähernd senkrecht zur Längsachse verlaufend die Achse der Bohrung 16 in gleichen Abständen umgeben. Diese Flächenabschnitte 31 würden jedoch beim Vortreiben in einem Markkanal einen zu großen Widerstand und damit einen zu großen Druck verursachen. Daher ist an den einander zugekehrten Flächen der Zähne 18 einer Spankammer 18 ein Anschliff vorgenommen, der sich aus Fig. 1 untere Hälfte und den Figuren 3 und 4 ergibt. Eine großflächige Anschlifffläche 36 ist im Anschnittbereich an der rückseitigen Fläche jedes Zahns 18 im Anschnittbereich vorgenommen. Sie führt zu einer Anspitzung des Anschnittbereichs, da die Fläche 36 einen größeren Winkel zur Längsachse hat als die übrige Rückseite der Zähne 18. Eine weitere Anschlifffläche 38 auf der Vorderseite am vorderen Ende der Zähne 18 ist flächenmäßig relativ klein. Beide Anschliffe 36, 38 führen zu einer Verringerung der Breite der Freifläche 40 der Zähne 18 im Anschnittbereich, wie aus Fig. 4 zu erkennen. Die Anschliffe 36, 38 sind so gelegt, daß sich insgesamt eine "Anspitzung" des Bohrkopfs 12 im Anschnittbereich ergibt mit der Ausbildung einer nahezu schneidenartigen scharfen Umrandung 42 des vorderen Endes der Bohrung 16. Da die scharfe Umrandung 42 jedoch Gefahr läuft, mit Hilfe eines Führungsspießes, der durch die Bohrung 16 hindurchgeführt ist, beschädigt zu werden, ist die Bohrung 16 am vorderen Ende konisch angesenkt, wie bei 44 zu erkennen, wodurch ein Spiel zwischen Bohrkopf 12 und Führungsspieß nicht ohne weiteres zu einer Beschädigung des scharfen Randes 42 führt. Der Anschliff 36, 38 und die Ansenkung 44 führen dazu, daß die vorderen Enden der Zähne 18 im Anschnittbereich zackenförmig vorstehen, wie bei 48 angedeutet, während der Rand 42 zwischen den Zacken 48 bogenförmig nach hinten verläuft (Fig. 3). Man erhält dadurch bei der Bearbeitung einen fräsenden Effekt.

Um den Unterschied zwischen den Figuren 1 und 2 und den Figuren 3 und 4 herauszustellen, sind das Werkzeug in Fign. 3 und 4 mit 10' und der Bohrkopf mit 12' bezeichnet.

Fig. 5 zeigt einen Schnitt durch einen Zahn 18 im Bereich der Nebenschneidkante 22. Dabei ist zu erkennen, daß die Spanfläche 50 als Hohlkehle gebildet ist. Der Spanwinkel a beträgt etwa 20 bis 22°. Die Freifläche an der Neben-Schneidkante 22 ist unterteilt in einen ersten Freiflächenabschnitt 52 sehr geringer Breite (siehe auch Fig. 1b) und einen zweiten etwas breiteren Freiflächenabschnitt 54. Ersterer hat einen Freiwinkel von etwa 13 bis 15 und der zweite von 15 bis 17°. Daran schließt sich die spiralförmige Rückfläche 56 des Zahns 18 an. Der Spanwinkel der Hauptschneidkante 20 ist ähnlich dem Spanwinkel a.

## Patentansprüche

1. Bohrkopf (12, 12') zum Aufbohren von Knochenkanälen mit einem Schaft (14), wobei der Bohrkopf (12, 12') folgende Merkmale aufweist:
eine mittige Bohrung (16) für einen Führungsdraht;
vier in 90°-Umfangsabstand angeordnete Zähne (18),
die im Anschnitt Hauptschneidkanten (20) bilden, die im Winkel zur Längsachse der Bohrung (16) verlaufen und
sich an die Hauptschneidkanten anschließende Nebenschneidkanten (22), die auf einem Zylinder (24) liegen, dessen Achse mit der Achse der Bohrung (16) zusammenfällt, wobei die Länge der Nebenschneidkanten (22) kleiner als der Durchmesser des Zylinders (24) ist, dadurch gekennzeichnet, daß
die Zähne (18) schraubenlinienförmig sind mit einem kleinen Drallwinkel;
die Hauptschneidkanten (20) einen Winkel von 45° zur Längsachse der Bohrung (16) aufweisen,
zwischen den Zähnen (18) gebildete Spankammern nutartig geformt sind mit gerundetem Grund (30), dessen radialer Abstand zur Bohrung (16) gering ist und nur so viel Material stehen läßt, daß eine ausreichende Stabilität gewährleistet ist und
im Anschnittbereich die die Spankammern (28) begrenzenden Flächen der Zähne (18) mit einem Anschliff (36, 38) so versehen sind, daß unter Anspitzung des Anschnitts ein schneidenartiger scharfer Rand (42) von den vorderen Enden der Zähne (18) und den dazwischen liegenden Spankammerflächen zum vorderen Ende der Bohrung (16) hin gebildet ist.

2. Bohrkopf nach Anspruch 1, dadurch gekennzeichnet, daß die Bohrung (16) am vorderen Ende eine Ansenkung (44) aufweist.

3. Bohrkopf nach Anspruch 1 oder 2, dadurch gekennzeichnet, daß die Außenkontur der Zähne (18) am hinteren Ende eine Rundung (26) zum Einspannschaft (14) hin aufweisen.

4. Bohrkopf nach einem der Ansprüche 1 bis 3, dadurch gekennzeichnet, daß die Spanflächen (50) eine durchgehende sich bis zum Anschliff (36, 38) erstreckende Hohlkehle aufweisen.

5. Bohrkopf nach einem der Ansprüche 1 bis 4, dadurch gekennzeichnet, daß eine erste sich an die Neben-Schneidkante (22) anschließende Freifläche (52) von geringerer Breite vorgesehen ist und einen ersten Freiwinkel bildet und die sich anschließende zweite Freifläche (54) größerer Breite einen größeren zweiten Freiwinkel aufweist.

6. Bohrkopf nach einem der Ansprüche 1 bis 5, dadurch gekennzeichnet, daß die Anschliffflächen (36, 38) plan sind.

7. Bohrkopf nach einem der Ansprüche 1 bis 6, dadurch gekennzeichnet, daß der Radius des Grundes (30) der Spankammern (28) gleich oder kleiner ist als der Außenradius des Schafts (14) bei entsprechendem Übergang von der Spankammer (28) zum Schaft (14).

## Claims

1. Drill head (12, 12') for boring out bone channels with a shank (14), wherein the drill head (12, 12') comprises the following features:
- a central bore (16) for a guide spike;
- four teeth (18) arranged at 90° intervals on the periphery,
- which form main cutting edges (20) in the starting cut, the cutting edges being angular to the longitudinal axis of bore (16) and
- secondary cutting edges (22) consecutive to the main cutting edges and lying on a cylinder (24) the axis of which coincides with the axis of the bore (16), wherein the length of the secondary cutting edges (22) is smaller than the diameter of the cylinder (24), characterized in that
- the teeth (18) are helically formed with a small angle of twist;
- the main cutting edges (20) have an angle of 45° to the longitudinal axis of the bore (16),
- the chip chambers between the teeth (18) are formed groove-like having a curved ground (30) being radially close to the bore (16) and leaving only enough material for providing sufficient stability, and
- the faces of the teeth (18) bordering the chip chambers (28) in the starting cut area are provided with a ground face (36, 38) such that sharpening the starting cut a relatively sharp edge (42) is formed, extending from the frontward ends of the teeth (18) over the chip chamber surfaces between them to the frontward end of the bore (16).

2. Drill head according to claim 1, characterized in that the bore (16) has a countersunk area (44) on the forward end.

3. Drill head according to claims 1 or 2, characterized in that the outside contour of the teeth (18) on the rear end has a curvature (26) toward the clamping shank (14).

4. Drill head according to one of the claims 1 to 3, characterized in that the chip faces (50) comprise a continuous concave surface extending to the ground face (36, 38).

5. Drill head according to one of claims 1 to 4, characterized in that a first flank surface (52) adjacent to the secondary cutting edge (22) has a smaller width and forms a first clearance angle, and the second flank surface (54) adjacent to the former has a larger second clearance angle.

6. Drill head according to one of claims 1 to 5, characterized in that the ground faces (36, 38) are planar.

7. Drill head according to one of the claims 1 to 6, characterized in that the radius of the ground (30) of the chip chambers (28) is less or equal to the outer radius of the shank (14) having an according transition from chip chamber (28) to shank (14).

## Revendications

1. Tête d'alésoir (12, 12') pour l'alésage de canaux d'os, comprenant une tige (14), la tête de l'alésoir (12, 12') présentant les particularités suivantes :
un trou central (16) destiné à un fil métallique de guidage ;
quatre dents (18) placées à des distances circonférentielles de 90°,
qui forment dans la zone d'attaque des arêtes principales de coupe (20) qui sont orientées de manière à inscrire un angle avec l'axe longitudinal du trou (16) et
des arêtes secondaires de coupe (22) qui se raccordent aux arêtes principales de coupe et qui sont situées sur un cylindre (24) dont l'axe coïncide avec l'axe du trou (16), la longueur des arêtes secondaires de coupe (22) étant plus faible que le diamètre du cylindre (24), caractérisée en ce que
les dents (18) sont hélicoïdales en ayant un faible angle d'hélice ;
les arêtes principales de coupe (20) inscrivent un angle de 45° avec l'axe longitudinal du trou (16),
des chambres à copeaux formées entre les dents (18) sont conformées à la manière de rainures ayant un fond arrondi (30) dont la distance radiale au trou (16) est faible et ne laisse subsister qu'une quantité suffisante de matière pour garantir assez de stabilité et
les surfaces des dents (18) qui délimitent les chambres à copeaux (28) comportent dans la zone d'attaque un affûtage (36, 38) tel que, sous l'effet d'un appointage de la zone d'attaque, un bord effilé à la manière d'une arête de coupe (42) est formé depuis les extrémités antérieures des dents (18) et les surfaces des chambres à copeaux qui sont situées entre ces dents jusqu'à l'extrémité antérieure du trou (16).

2. Tête d'alésoir selon la revendication 1, caractérisée en ce que le trou (16) comporte un chanfrein (44) à l'extrémité antérieure.

3. Tête d'alésoir selon la revendication 1 ou 2, caractérisée en ce que le contour extérieur des dents (18) présente à l'extrémité arrière un arrondi (26) aboutissant à la tige de fixation (14).

4. Tête d'alésoir selon l'une des revendications 1 à 3, caractérisée en ce que les faces de coupe (50) présentent un congé continu qui se prolonge jusqu'à l'affûtage (36, 38).

5. Tête d'alésoir selon l'une des revendications 1 à 4, caractérisée en ce qu'une première face de dépouille (52) qui se raccorde à l'arête secondaire de coupe (22) et qui a une faible largeur est prévue et forme un premier angle de dépouille et la deuxième face de dépouille (54) qui fait suite et qui a une plus grande largeur présente un deuxième angle de dépouille qui est plus grand.

6. Tête d'alésoir selon l'une des revendications 1 à 5, caractérisée en ce que les surfaces d'affûtage (36, 38) sont planes.

7. Tête d'alésoir selon l'une des revendications 1 à 6, caractérisée en ce que le rayon du fond (30) des chambres à copeaux (28) est égal ou inférieur au rayon extérieur de la tige (14) avec une transition correspondante de la chambre à copeaux (28) à la tige (14).
